# FASCICULE DE BREVET EUROPEEN

(11) **EP 0 532 387 B1**
(45) Date de publication et mention de la délivrance du brevet: **28.12.1994**
(21) Numéro de dépôt: 92402396.3
(22) Date de dépôt: 03.09.1992
(51) Int. Cl.: F25J 3/02, C07C 7/04, C07C 9/04

(54) **Procédé et installation de production de monoxyde de carbone et d'hydrogène**
Verfahren und Vorrichtung für die Herstellung von Kohlenoxid und Wasserstoff
Process and installation for the production of carbon monoxide and hydrogen

(30) Priorité: 11.09.1991 FR 9111197
(43) Date de publication de la demande: 17.03.1993
(73) Titulaire: L'AIR LIQUIDE, SOCIETE ANONYME POUR L'ETUDE ET L'EXPLOITATION DES PROCEDES GEORGES CLAUDE, 75321 Paris Cédex 07 (FR)
(72) Inventeur: Billy, Jean, F-94420 Le Plessis Trevise (FR)
(74) Mandataire: Le Moenner, Gabriel

(56) Documents cités:
- EP-A- 0 317 851
- DE-C- 621 453
- FR-A- 2 353 819

## Description

La présente invention est relative à un procédé de production de monoxyde de carbone et d'hydrogène à partir d'un mélange gazeux comprenant essentiellement ces deux corps et du méthane, du type dans lequel : le mélange gazeux de départ est lavé au méthane liquide dans une première colonne pour fournir de l'hydrogène gazeux et un liquide de lavage ; l'hydrogène dissous dans le liquide de lavage est séparé de celui-ci dans une deuxième colonne, et le liquide restant est séparé par distillation dans une troisième colonne pour fournir du monoxyde de carbone de production en tête de cette troisième colonne.

Dans la technique classique, telle que connue, par exemple, du document FR-A-2 353 819, afin de n'utiliser qu'un compresseur unique pour l'ensemble de l'installation, le monoxyde de carbone est soutiré sous forme gazeuse de la troisième colonne et sert de gaz de cycle pour assurer le chauffage et/ou le refroidissement des trois colonnes.

Cette utilisation d'un cycle monoxyde de carbone ouvert présente les inconvénients suivants :
- d'une part, la compression du monoxyde de carbone pose quelques difficultés de réalisation dues notamment à la potentialité, au cours de la compression, de réactions chimiques de décomposition de la molécule de monoxyde de carbone conduisant à la formation de composés (carbone, dioxyde de carbone, ...) qui réduisent la fiabilité de l'unité de séparation et de la compression ;
- d'autre part, la mise en oeuvre d'un cycle de monoxyde de carbone induit dans l'unité une masse résidente de ce gaz, sous forme liquide et gazeuse, qui est source de risque en cas d'accident.

L'invention a pour but, tout en conservant un compresseur unique pour l'ensemble de l'installation, d'éviter ces inconvénients, c'est-à-dire d'éliminer les risques associés à la compression du monoxyde de carbone gazeux et de réduire les risques potentiels associés à la masse résidente de monoxyde de carbone gazeux et liquide.

Le DE-C-621 453 décrit aussi un procédé de production de monoxyde de carbone à partir d'un mélange gazeux contenant essentiellement du monoxyde de carbone de l'hydrogène et du méthane, dans lequel ou chauffe et/ou refroidit deux colonnes au moyen d'un cycle frigorifique fermé à azote. Le monoxyde de carbone est soutiré sous forme gazeuse dans la deuxième colonne.

A cet effet, l'invention a pour objet un procédé du type précité, caractérisé en ce qu'on chauffe et/ou on refroidit chaque colonne au moyen d'un cycle frigorifique fermé, et en ce qu'on soutire le monoxyde de carbone de production de la troisième colonne sous forme liquide, puis on l'amène par pompe à la pression d'utilisation et on le réchauffe sous cette pression d'utilisation.

Le cycle frigorifique peut notamment être un cycle azote.

L'invention a également pour objet une installation destinée à la mise en oeuvre d'un tel procédé. Cette installation, du type comprenant : une première colonne munie en cuve d'une entrée dudit mélange gazeux et en tête d'une entrée de méthane liquide ; une deuxième colonne munie en cuve d'un vaporiseur et en tête d'une entrée de liquide reliée à la cuve de la première colonne ; une troisième colonne munie en cuve d'un vaporiseur, en tête de moyens de création de reflux et de moyens de soutirage de monoxyde de carbone, et en un point intermédiaire d'une entrée reliée à la cuve de la deuxième colonne ; et un cycle frigorifique assurant le chauffage et/ou le refroidissement des trois colonnes, est caractérisée en ce que le cycle frigorifique est un cycle fermé, et en ce que les moyens de soutirage de monoxyde de carbone sont des moyens de soutirage de liquide reliés à l'aspiration d'une pompe, l'installation comprenant des moyens de réchauffement du fluide refoulé par cette pompe.

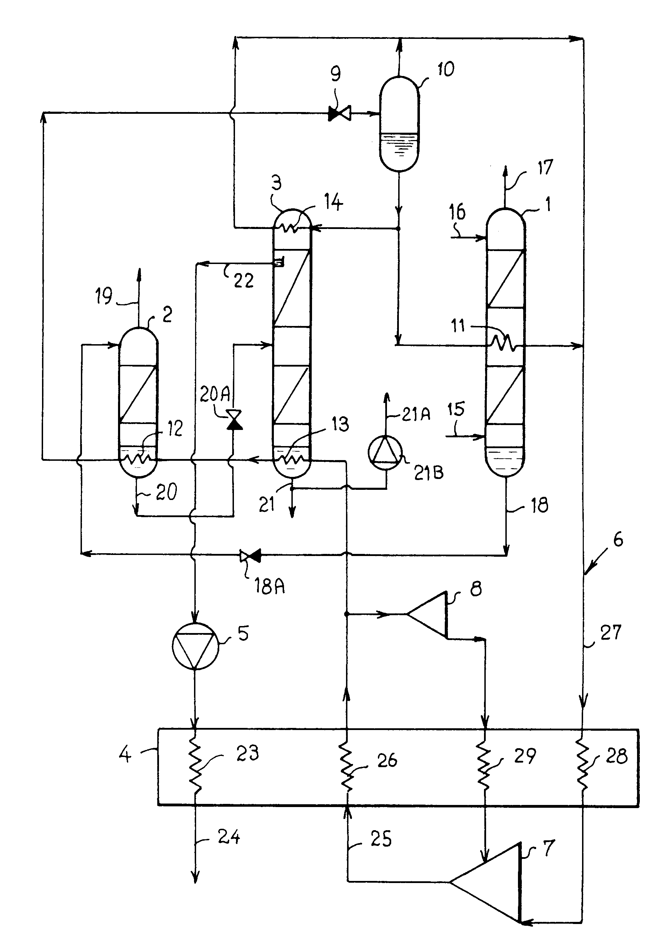

Un exemple de mise en oeuvre de l'invention va maintenant être décrit en regard du dessin annexé, dont la Figure unique représente schématiquement une installation de production de monoxyde de carbone et d'hydrogène conforme à l'invention.

L'installation représentée au dessin est destinée à produire du monoxyde de carbone (CO) gazeux sous pression et de l'hydrogène gazeux également sous pression, à partir d'un mélange gazeux initial contenant essentiellement du monoxyde de carbone, de l'hydrogène et du méthane. Ce mélange, qui est par exemple un gaz de réformage ou d'oxydation partielle d'hydrocarbures, préalablement desséché et décarbonaté, contient généralement en faibles quantités des impuretés lourdes CₓH_{y} et des impuretés plus légères que le CO telles que l'azote et l'argon.

L'installation comprend essentiellement une première colonne, ou colonne de lavage, 1, une deuxième colonne 2, une troisième colonne, ou colonne de distillation, 3, un échangeur de chaleur 4 du type indirect et à circulation à contre-courant des fluides mis en relation d'échange thermique, une pompe 5, et un cycle frigorifique 6. Ce cycle 6 comprend un compresseur 7, une turbine de détente 8, une vanne de détente 9 et un séparateur de phases 10.

Pour assurer le chauffage et/ou le refroidissement des trois colonnes 1 à 3 au moyen du cycle frigorifique 6, la colonne 1 comporte à un niveau intermédiaire un condenseur 11, la colonne 2 comporte en cuve un vaporiseur 12, et la colonne 3 comporte en cuve un vaporiseur 13 et en tête un condenseur 14.

Le mélange gazeux initial, introduit en cuve de la colonne 1, via une conduite 15, sous une pression par exemple de l'ordre de 15 à 40 bars absolus, est lavé dans cette colonne au moyen de méthane liquide introduit en tête de colonne via une conduite 16, une réfrigération intermédiaire de la colonne étant assurée par le condenseur 11.

Un courant d'hydrogène gazeux de production, sous pression, est soutiré en tête de la colonne 1 via une conduite 17, tandis que le liquide de cuve, essentiellement constitué de monoxyde de carbone, de méthane et d'hydrogène, est envoyé, via une conduite 18 équipée d'une vanne de détente 18A, en tête de la colonne 2. Cette dernière étant chauffée en cuve par le vaporiseur 12, l'hydrogène dissous est séparé du liquide dans cette deuxième colonne, sous une pression de l'ordre de 5 à 10 bars, et est évacué en tête via une conduite 19.

Le liquide de cuve de la colonne 2, constitué essentiellement de méthane et de monoxyde de carbone, est introduit, via une conduite 20 équipée d'une vanne de détente 20A, en un point intermédiaire de la colonne 3. Cette dernière, chauffée en cuve par le vaporiseur 13 et refroidie en tête par le condenseur 14, sépare les deux constituants du liquide par distillation sous une pression de l'ordre de 1 à 2,5 bars. Du méthane liquide contenant les impuretés lourdes est soutiré en cuve de la colonne 3 via une conduite 21, et du monoxyde de carbone liquide contenant des impuretés légères est soutiré en tête de la même colonne via une conduite 22.

Tandis que le méthane liquide est en partie purgé et en partie recyclé, via une conduite 21A équipée d'une pompe 21B, dans la conduite 16 qui alimente la tête de la colonne 1, le monoxyde de carbone liquide est amené par la pompe 5 à la pression d'utilisation désirée, puis réchauffé sous cette pression dans des passages 23 de l'échangeur de chaleur 4, d'où il ressort sous forme de produit gazeux via une conduite 24.

On décrira maintenant le cycle frigorifique 6, qui est un cycle azote fermé, c'est-à-dire dont le fluide de cycle est de l'azote et qui est entièrement séparé des circuits de fluides précités.

L'azote de cycle, comprimé par le compresseur 7 dans une conduite haute pression 25, est refroidi dans des passages 26 de l'échangeur 4 puis condensé dans des vaporiseurs 13 puis 12. L'azote liquide haute pression est détendu dans la vanne de détente 9 et séparé en deux phases dans le séparateur 10. L'azote gazeux basse pression issu de ce séparateur est renvoyé via une conduite 27 et des passages 28 de l'échangeur 4 à l'aspiration basse pression du compresseur, tandis que l'azote liquide basse pression est vaporisé dans les condenseurs 11 et 14, l'azote gazeux résultant de ces vaporisations étant lui aussi envoyé dans la conduite 27. Une fraction de l'azote gazeux haute pression sortant des passages 26 est détendue dans la turbine 8 à une pression intermédiaire puis réchauffée dans des passages 29 de l'échangeur 4 et renvoyé à un étage intermédiaire du compresseur 7.

L'installation décrite ci-dessus permet de réduire à un minimum la masse résidente de monoxyde de carbone, notamment du fait que la masse de fluide de cycle stockée sous forme liquide dans le séparateur 10, généralement utilisée comme réserve de froid, est constituée d'azote.

## Revendications

1. Procédé de production de monoxyde de carbone et d'hydrogène à partir d'un mélange gazeux comprenant essentiellement ces deux corps et du méthane, du type dans lequel : le mélange gazeux de départ est lavé au méthane liquide dans une première colonne (1) pour fournir de l'hydrogène gazeux et un liquide de lavage ; l'hydrogène dissous dans le liquide de lavage est séparé de celui-ci dans une deuxième colonne (2), et le liquide restant est séparé par distillation dans une troisième colonne (3) pour fournir du monoxyde de carbone de production en tête de cette troisième colonne, caractérisé en ce qu'on chauffe et/ou on refroidit chaque colonne (1, 2, 3) au moyen d'un cycle frigorifique fermé, et en ce qu'on soutire le monoxyde de carbone de production de la troisième colonne (3) sous forme liquide, puis on l'amène par pompe (5) à la pression d'utilisation et on le réchauffe sous cette pression d'utilisation.

2. Procédé suivant la revendication 1, caractérisé en ce que le cycle frigorifique est un cycle azote.

3. Installation de production de monoxyde de carbone et d'hydrogène à partir d'un mélange gazeux comprenant essentiellement ces deux corps et du méthane, du type comprenant : une première colonne (1) munie en cuve d'une entrée (15) dudit mélange gazeux et en tête d'une entrée (16) de méthane liquide ; une deuxième colonne (2) munie en cuve d'un vaporiseur (12) et en tête d'une entrée de liquide reliée à la cuve de la première colonne (1) ; une troisième colonne (3) munie en cuve d'un vaporiseur (13), en tête de moyens (14) de création de reflux et de moyens (22) de soutirage de monoxyde de carbone, et en un point intermédiaire d'une entrée reliée à la cuve de la deuxième colonne (2) ; et un cycle frigorifique (6) assurant le chauffage et/ou le refroidissement des trois colonnes (1 à 3), caractérisée en ce que le cycle frigorifique (6) est un cycle fermé, et en ce que les moyens (22) de soutirage de monoxyde de carbone sont des moyens de soutirage de liquide reliés à l'aspiration d'une pompe (5), l'installation comprenant des moyens (4) de réchauffement du fluide refoulé par cette pompe (5).

4. Installation suivant la revendication 3, caractérisée en ce que le cycle frigorifique est un cycle azote.

## Claims

1. Process for producing carbon monoxide and hydrogen from a gaseous mixture comprising essentially these two substances and methane, of the type in which: the initial gas mixture is washed in liquid methane in a first column (1) so as to provide gaseous hydrogen and a washing liquid; the hydrogen dissolved in the washing liquid is separated from the latter in a second column (2), and the remaining liquid is separated by distillation in a third column (3) so as to provide producer carbon monoxide at the head of this third column, characterised in that each column (1, 2, 3) is heated and/or cooled by means of a closed refrigeration cycle, and in that the producer carbon monoxide is drawn off from the third column (3) in liquid form, and then brought by means of a pump (5) to the utilisation pressure and heated at this utilisation pressure.

2. Process according to Claim 1, characterised in that the refrigeration cycle is a nitrogen cycle.

3. Installation for producing carbon monoxide and hydrogen from a gaseous mixture essentially comprising these two substances and methane, of the type comprising: a first column (1) equipped at its base with an inlet (15) for the said gaseous mixture and at its head with a liquid-methane inlet (16); a second column (2) equipped at its base with a vaporiser (12) and at its head with a liquid inlet connected to the base of the first column (1); a third column (3) equipped at its base with a vaporiser (13), at its head with means (14) for creating reflux and with means (22) for taking off carbon monoxide and, at an intermediate point, an inlet connected to the base of the second column (2); and a refrigeration cycle (6) ensuring the heating and/or cooling of the three columns (1 to 3), characterised in that the refrigeration cycle (6) is a closed cycle, and in that the means (22) for taking off carbon monoxide are means for taking off liquid connected to the intake of a pump (5), the installation comprising means (4) for heating the fluid discharged by this pump (5).

4. Installation according to Claim 3, characterised in that the refrigeration cycle is a nitrogen cycle.

## Patentansprüche

1. Verfahren zur Gewinnung von Kohlenstoffmonoxid und Wasserstoff aus einem Gasgemisch, das im wesentlichen diese beiden Bestandteile und Methan enthält, umfassend die Schritte: Waschen des Ausgangsgasgemisches mit flüssigem Methan in einer ersten Säule (1), um gasförmigen Wasserstoff und eine Waschflüssigkeit zu erhalten; Trennen des in der Waschflüssigkeit gelösten Wassers von dieser in einer zweiten Säule (2) und Trennen der verbleibenden Flüssigkeit durch Destillation in einer dritten Säule (3), um Produktionskohlenmonoxid am Kopf dieser dritten Säule zu erhalten, **dadurch gekennzeichnet,** daß jede Säule (1, 2, 3) mittels eines geschlossenen Kühlkreislaufs erwärmt und/oder abgekühlt wird, und daß das in der dritten Säule (3) gewonnene Kohlenmonoxid in flüssiger Form abgezogen wird, woraufhin das Kohlenmonoxid durch eine Pumpe (3) auf den Verwendungsdruck gebracht und unter diesem Verwendungsdruck wiedererwärmt wird.

2. Verfahren nach Anspruch 1, dadurch gekennzeichnet, daß der Kühlkreislauf ein Stickstoffkreislauf ist.

3. Anlage zur Gewinnung von Kohlenstoffmonoxid und Wasserstoff aus einem Gasgemisch, das im wesentlichen diese beiden Bestandteile und Methan enthält, mit: einer ersten Säule (1), die am Fußpunkt mit einem Einlaß (15) für das Gasgemisch und am Kopf mit einem Einlaß (16) für das flüssige Methan versehen ist; einer zweiten Säule (2), die am Fußpunkt mit einem Verdampfer (12) und am Kopf mit einem Flüssigkeitseinlaß versehen ist, der mit dem Fußpunkt der ersten Säule (1) verbunden ist; einer dritten Säule (3), die am Fußpunkt mit einem Verdampfer (13), am Kopf mit einer Einrichtung (14) zur Erzeugung des Rückflusses und mit einer Einrichtung (22) zum Abziehen des Kohlenstoffmonoxids und an einem Zwischenpunkt mit einem Einlaß versehen ist, der mit dem Fußpunkt der zweiten Säule (2) verbunden ist; und einem Kühlkreislauf (6), der das Erwärmen und/oder Kühlen der drei Säulen (1 bis 3) sicherstellt, dadurch gekennzeichnet, daß der Kühlkreislauf (6) ein geschlossener Kreislauf ist, und daß die Einrichtung (22) zum Abziehen des Kohlenstoffmonoxids eine Einrichtung zum Abziehen der Flüssigkeit ist, die mit der Saugseite einer Pumpe (5) verbunden ist, wobei die Anlage eine Einrichtung (4) zum Wiedererwärmen der von dieser Pumpe (5) ausgestoßenen Flüssigkeit umfaßt.

4. Anlage nach Anspruch 3, dadurch gekennzeichnet, daß der Kühlkreislauf ein Stickstoffkreislauf ist.
